# EUROPEAN PATENT APPLICATION

(11) **EP 4 825 372 A1**
(43) Date of publication of application: **21.10.2026**
(21) Application number: 25745362.1
(22) Date of filing: 23.01.2025
(51) Int. Cl.: A61K 9/16, A61K 38/26, A61P 3/04, A61P 3/10, A61P 9/12, A61P 1/16, A61P 25/28, A61K 9/00

(54) **MICROSPHERES COMPRISING GLP-1 RA-BASED PEPTIDES, METHOD FOR PREPARING SAME, AND PHARMACEUTICAL COMPOSITION COMPRISING SAME**

(30) Priority: 26.01.2024 KR 20240012693; 22.01.2025 KR 20250009554
(71) Applicant: Aulbio Co., Ltd., Seongnam-si, Gyeonggi-do 13105 (KR)
(72) Inventor: AN, Tae Kun, Yongin-si, Gyeonggi-do 17003 (KR); KIM, A Ram, Seoul 05667 (KR); KIM, Seo A, Gimpo-si, Gyeonggi-do 10089 (KR); PARK, Ji Won, Hwaseong-si, Gyeonggi-do 18266 (KR); KIM, Cherng Ju, Beaumont, California 92223 (US)
(74) Representative: dompatent
(86) International application number: PCT/KR2025/001341
(87) International publication number: WO 2025/159538

(57) **Abstract**

The present invention relates to microspheres comprising GLP-1 RA-based peptides, a method for preparing the same, and a pharmaceutical composition comprising the same. Provided are microspheres having excellent bioavailability, comprising GLP-1 RA-based peptides, a bioavailability enhancer, and a biocompatible polymer.

## Description

### [Technical Field]

This application claims the benefit of priorities based on Korean Patent Application No. 10-2024-0012693 filed on January 26, 2024, and Korean Patent Application No. 10-2025-0009554 filed on January 22, 2025, and the entire contents of which are incorporated as part of this specification.

The present invention relates to microspheres comprising GLP-1 receptor agonist (RA)-based peptides, a method for preparing the same, and a pharmaceutical composition comprising the same. More specifically, it relates to microspheres comprising GLP-1 RA-based peptides, a bioavailability enhancer, and a biocompatible polymer; a method for preparing the same; and a pharmaceutical composition comprising the same.

### [Background Art]

Obesity is a disease accompanied by metabolic dysregulation, particularly abnormal glucose metabolism, and characteristic organ complications, and is a chronic disease that requires long-term drug treatment. Various anti-obesity therapeutics, including human insulin and other GLP-1 RA-based peptides, are commercially available for the treatment of obesity.

For example, semaglutide or tirzepatide is one of the therapeutics used for the treatment of obesity. Semaglutide or tirzepatide is once-weekly subcutaneous injection therapies and approved and marketed as drugs for improving weight control in obese patients and for controlling blood sugar levels in diabetic patients.

The high dosing frequency of these injections causes significant patients discomfort, thereby reducing medication compliance. Long-acting injections of GLP-1 RA-based peptides are being continuously researched to reduce the dosing frequency of therapeutics for obese patients.

In this regard, various formulation technologies have been utilized to provide sustained and controlled release of pharmacologically active substances. Among these, the injectable formulations comprising long-acting microspheres are provided in the form of microspheres in which the pharmacologically active substances are encapsulated within a polymer matrix, and are designed so that the drug can be uniformly released from the microspheres upon subcutaneous or intramuscular injection.

To ensure that release formulations using such long-acting microspheres actually lead to the effects of extending the dosing interval and reducing the administration dose, it is necessary to not only control release efficiently but also ensure bioavailability.

However, when peptide drugs such as semaglutide and tirzepatide are developed as long-acting injections to reduce dosing frequency, a problem occurs in that their bioavailability is significantly lowered during long-term storage *in vivo* due to the nature of the peptides.

To address these problems, research is ongoing to develop long-acting injections with high *in vivo* bioavailability over extended periods, and there is a need to develop injectable formulations comprising long-acting microspheres capable of enhancing bioavailability.

### [Prior Art Document]

### [Patent Documents]

Korean Laid-open Patent Application No. 10-2021-0147413

### [Disclosure]

### [Technical Problem]

In order to solve the aforementioned problems of the prior art, it is an object of the present invention to provide microspheres comprising GLP-1 RA-based peptides by using a bioavailability enhancer as an additive.

It is another object of the preseent invention to provide microspheres with excellent bioavailability and a method for preparing the same.

It is another object of the preseent invention to provide a pharmaceutical composition for preventing or treating diseases such as obesity and diabetes, comprising the microspheres.

### [Technical Solution]

In order to solve the aforementioned problems,
the present invention provides microspheres comprising GLP-1 RA-based peptides; a bioavailability enhancer; and a biocompatible polymer.

The present invention provides the microspheres, wherein the GLP-1 RA-based peptides are one or more selected from the group consisting of a GLP-1 receptor agonist, a GIP/GLP-1 receptor dual agonist, and a GLP-1/GIP/GCG receptor triple agonist.

The present invention provides the microspheres, wherein the bioavailability enhancer is one or more selected from the group consisting of a surfactant, a polyhydric alcohol, an amino acid, and a fatty acid.

The present invention provides the microspheres, wherein the bioavailability enhancer is included in an amount of 0.05% to 50% by weight based on the total weight of the microspheres.

The present invention provides the microspheres, wherein the surfactant is one or more selected from poloxamer 188, poloxamer 407, polysorbate 20, polysorbate 60, polysorbate 80, polyoxyethylene oleyl ether, polyoxyethylene cetyl ether, polyoxyethylene stearyl ether, and polyoxyethylene lauryl ether.

The present invention provides the microspheres, wherein the polyhydric alcohol is one or more selected from ethylene glycol, propylene glycol, polyethylene glycol, glycerol, erythritol, threitol, xylitol, ribitol, mannitol, sorbitol, and maltitol.

The present invention provides the microspheres, wherein the amino acid is one or more selected from lysine, arginine, aspartic acid, histidine, alanine, phenylalanine, serine, glutamine, glycine, tryptophan, valine, leucine, isoleucine, cysteine, methionine, and proline.

The present invention provides the microspheres, wherein the fatty acid is one or more selected from stearic acid, palmitic acid, lauric acid, myristic acid, oleic acid, and arachidonic acid.

The present invention provides the microspheres, wherein the GLP-1 RA-based peptides are selected from semaglutide, tirzepatide, retatrutide, exenatide, liraglutide, lixisenatide, albiglutide, dulaglutide, cotadutide, taspoglutide, pemvidutide, survodutide, orforglipron, mazdutide, MariTide, ecnoglutide, cagrilintide/semaglutide, efpeglenatide, dapiglutide, pramlintide, and combinations thereof.

The present invention provides the microspheres, wherein the GLP-1 RA-based peptides are included in an amount of 0.1% to 50% by weight based on the total weight of the microspheres.

The present invention provides the microspheres, wherein the biocompatible polymer is any one or more selected from polylactic acid, polylactide, polylactic-co-glycolic acid, polylactide-co-glycolide (PLGA), polyphosphazine, polyiminocarbonate, polyphosphoester, polyanhydride, polyorthoester, copolymer of lactic acid and caprolactone, polycaprolactone, polyhydroxyvalerate, polyhydroxybutyrate, polyamino acid, and copolymer of lactic acid and amino acid.

The present invention provides the microspheres, wherein the biocompatible polymer is included in an amount of 50% to 99.9% by weight based on the total weight of the microspheres.

In addition, the present invention provides a method for preparing microspheres, comprising the steps of: (a1) dissolving a biocompatible polymer in one or more solvents to prepare an oil phase (O); (b1) dissolving or dispersing GLP-1 RA-based peptides and a bioavailability enhancer in a solvent to prepare an inner water phase (W1); (c1) dispersing the inner water phase (W1) into the oil phase (O) to prepare a water-in-oil (W1/O) type emulsion; (d1) solidifying microspheres by dispersing the water-in-oil (W1/O) type dispersed phase prepared in the step (c1) into an outer continuous phase (W2) to prepare a water-in-oil-in-water (W1/O/W2) type emulsion solution; and (e1) removing the solvent.

In addition, the present invention provides a method for preparing microspheres, comprising the steps of: (a2) dissolving a biocompatible polymer and a bioavailability enhancer in one or more solvents to prepare an oil phase (O); (b2) dissolving or dispersing GLP-1 RA-based peptides in a solvent to prepare an inner water phase (W1); (c2) dispersing the inner water phase (W1) into the oil phase (O) to prepare a water-in-oil (W1/O) type emulsion; (d2) solidifying microspheres by dispersing the water-in-oil (W1/O) type dispersed phase prepared in the step (c2) into an outer continuous phase (W2) to prepare a water-in-oil-in-water (W1/O/W2) type emulsion solution; and (e2) removing the solvent.

In addition, the present invention provides a method for preparing microspheres, comprising the steps of: (a3) dissolving a biocompatible polymer and a bioavailability enhancer in one or more solvents to prepare an oil phase (O); (b3) dissolving or dispersing GLP-1 RA-based peptides and a bioavailability enhancer in a solvent to prepare an inner water phase (W1); (c3) dispersing the inner water phase (W1) into the oil phase (O) to prepare a water-in-oil (W1/O) type emulsion; (d3) solidifying microspheres by dispersing the water-in-oil (W1/O) type dispersed phase prepared in the step (c3) into an outer continuous phase (W2) to prepare a water-in-oil-in-water (W1/O/W2) type emulsion solution; and (e3) removing the solvent.

In addition, the present invention provides a pharmaceutical composition for preventing or treating obesity, diabetes, hypertension, hyperlipidemia, non-alcoholic steatohepatitis, metabolic dysfunction-associated steatohepatitis, cardiovascular disease, or neurodegenerative disease, comprising the microspheres.

The present invention provides the pharmaceutical composition, which is for subcutaneous or intramuscular injection.

### [Advantageous Effects]

The microspheres according to the present invention may increase the bioavailability of GLP-1 RA-based peptides, thereby providing the effects of reducing dosing frequency and reducing administration dose.

In addition, the microspheres comprising GLP-1 RA-based peptides, prepared by the method for preparing microspheres according to the present invention, may increase bioavailability by containing a bioavailability enhancer, and provides the effects of reducing dosing frequency and reducing administration dose.

In addition, the pharmaceutical composition according to the present invention provides an excellent effect on preventing or treating obesity or diabetes by comprising the microspheres.

### [Best Mode]

Hereinafter, the present invention will be described in more detail.

Unless defined otherwise, all technical terms used in the present invention have the same meaning as commonly understood by those skilled in the relevant art of the present invention. In addition, those similar or equivalent to the preferred methods or samples described in the present invention are also included in the scope of the present invention. The contents of all publications cited as references in this specification are incorporated in this specification by reference in their entirety.

Among conventional therapeutics used for the treatment of obesity, semaglutide or tirzepatide is once-weekly subcutaneous injection therapies and has problems in that it causes significant patients discomfort due to the high dosing frequency and also reduces medication compliance.

To solve these problems, the present inventors have developed a double emulsion method capable of incorporating a bioavailability enhancer as an additive into long-acting microspheres comprising GLP-1 RA-based peptides, and thus have completed microspheres capable of achieving high bioavailability and minimizing the administration dose.

The microspheres according to one embodiment of the present invention may comprise GLP-1 RA-based peptides; a bioavailability enhancer; and a biocompatible polymer.

The microspheres are characterized by comprising the bioavailability enhancer therein as an additive, in addition to the GLP-1 RA-based peptides and the biocompatible polymer.

In one embodiment of the present invention, the bioavailability enhancer may include one or more selected from the group consisting of a surfactant, a polyhydric alcohol, an amino acid, and a fatty acid.

In one embodiment of the present invention, the surfactant may be one or more selected from poloxamer 188, poloxamer 407, polysorbate 20, polysorbate 60, polysorbate 80, polyoxyethylene oleyl ether, polyoxyethylene cetyl ether, polyoxyethylene stearyl ether, and polyoxyethylene lauryl ether. More specifically, the surfactant may be poloxamer 188, poloxamer 407, polyoxyethylene oleyl ether, polyoxyethylene cetyl ether, polyoxyethylene stearyl ether, or polyoxyethylene lauryl ether, but is not limited only to these examples.

In one embodiment of the present invention, the polyhydric alcohol may be one or more selected from ethylene glycol, propylene glycol, polyethylene glycol, glycerol, erythritol, threitol, xylitol, ribitol, mannitol, sorbitol, and maltitol. More specifically, the polyhydric alcohol may be ethylene glycol, propylene glycol, or polyethylene glycol, but is not limited only to these examples.

In one embodiment of the present invention, the amino acid may be one or more selected from lysine, arginine, aspartic acid, histidine, alanine, phenylalanine, serine, glutamine, glycine, tryptophan, valine, leucine, isoleucine, cysteine, methionine, and proline, but is not limited only to these examples.

In one embodiment of the present invention, the fatty acid may be one or more selected from stearic acid, palmitic acid, lauric acid, myristic acid, oleic acid, and arachidonic acid, but is not limited only to these examples.

In one embodiment of the present invention, the microspheres may comprise the bioavailability enhancer in an amount of 0.05% to 50% by weight based on the total weight of the microspheres. More specifically, the content of the bioavailability enhancer comprised in the microspheres may be 0.05% by weight or more, 0.1% by weight or more, 0.5% by weight or more, 1.0% by weight or more, 1.5% by weight or more, 2.0% by weight or more, 2.5% by weight or more, 3.0% by weight or more, 3.5% by weight or more, 4.0% by weight or more, 4.5% by weight or more, 5.0% by weight or more, 5.5% by weight or more, 6.0% by weight or more, 6.5% by weight or more, 7.0% by weight or more, 7.5% by weight or more, 8.0% by weight or more, 8.5% by weight or more, 9.0% by weight or more, 9.5% by weight or more, or 10.0% by weight or more, or may be 50% by weight or less, 49.5% by weight or less, 49.0% by weight or less, 48.5% by weight or less, 48.0% by weight or less, 47.5% by weight or less, 47.0% by weight or less, 46.5% by weight or less, 46.0% by weight or less, 45.5% by weight or less, 45.0% by weight or less, 44.5% by weight or less, 44.0% by weight or less, 43.5% by weight or less, 43.0% by weight or less, 42.5% by weight or less, 42.0% by weight or less, 41.5% by weight or less, 41.0% by weight or less, 40.5% by weight or less, or 40.0% by weight or less, based on the total weight of the microspheres.

In one embodiment of the present invention, the GLP-1 RA-based peptides may be one or more selected from the group consisting of a GLP-1 receptor agonist, a GIP/GLP-1 receptor dual agonist, and a GLP-1/GIP/GCG receptor triple agonist.

In one embodiment of the present invention, the GLP-1 RA-based peptides may be selected from semaglutide, tirzepatide, retatrutide, exenatide, liraglutide, lixisenatide, albiglutide, dulaglutide, cotadutide, taspoglutide, pemvidutide, survodutide, orforglipron, mazdutide, MariTide, ecnoglutide, dapiglutide, cagrilintide/semaglutide, efpeglenatide, pramlintide, and combinations thereof.

The GLP-1 RA-based peptides may be prepared in various forms, and for example, may be amorphous or crystalline.

In one embodiment of the present invention, the GLP-1 RA-based peptides may be included in an amount of 0.1% to 50% by weight based on the total weight of the microspheres. More specifically, the content of the GLP-1 RA-based peptides may be 0.1% by weight or more, 0.5% by weight or more, 1.0% by weight or more, 1.5% by weight or more, 2.0% by weight or more, 2.5% by weight or more, 3.0% by weight or more, 3.5% by weight or more, 4.0% by weight or more, 4.5% by weight or more, 5.0% by weight or more, 5.5% by weight or more, 6.0% by weight or more, 6.5% by weight or more, 7.0% by weight or more, 7.5% by weight or more, 8.0% by weight or more, 8.5% by weight or more, 9.0% by weight or more, 9.5% by weight or more, or 10.0% by weight or more, or may be 50% by weight or less, 49.5% by weight or less, 49.0% by weight or less, 48.5% by weight or less, 48.0% by weight or less, 47.5% by weight or less, 47.0% by weight or less, 46.5% by weight or less, 46.0% by weight or less, 45.5% by weight or less, 45.0% by weight or less, 44.5% by weight or less, 44.0% by weight or less, 43.5% by weight or less, 43.0% by weight or less, 42.5% by weight or less, 42.0% by weight or less, 41.5% by weight or less, 41.0% by weight or less, 40.5% by weight or less, or 40.0% by weight or less, based on the total weight of the microspheres.

If the content of the GLP-1 RA-based peptides exceeds 50% by weight based on the total weight of the microspheres, a problem may arise in that the initial release amount of the GLP-1 RA-based peptides is excessively high in an *in vivo* environment, and thus, the blood concentration of the drug rapidly increases. In addition, if the content of the GLP-1 RA-based peptides is less than 0.1% by weight based on the total weight of the microspheres, there may be a problem that the proportion of the biocompatible polymer becomes relatively high, and thus, the *in vivo* dose becomes excessive, making administration difficult.

The microspheres according to one embodiment of the present invention mean microspheres in which the GLP-1 RA-based peptides are encapsulated in the microsppheres prepared using a biocompatible polymer, and are referred to simply as GLP-1 RA-based peptide-containing microspheres, GLP-1 RA-based peptide microspheres, microspheres, or the like, in this specification.

In one embodiment of the present invention, the biocompatible polymer means a polymer that has been guaranteed to be safe *in vivo* by not causing high cytotoxicity and inflammatory responses when administered into a living body, and is also referred to simply as a polymer in this specification.

The biocompatible polymer may be any one or more selected from polylactic acid, polylactide, polylactic-co-glycolic acid, polylactide-co-glycolide (PLGA), polyphosphazine, polyiminocarbonate, polyphosphoester, polyanhydride, polyorthoester, copolymer of lactic acid and caprolactone, polycaprolactone, polyhydroxyvalerate, polyhydroxybutyrate, polyamino acid, and copolymer of lactic acid and amino acid.

In one embodiment of the present invention, the biocompatible polymer may have an intrinsic viscosity of 0.1 dL/g to 1.9 dL/g. The intrinsic viscosity refers to a value measured at a concentration of 0.05% (w/v) to 3% (w/v) in chloroform at 25°C using an Ubbelohde viscometer.

If the biocompatible polymer has an intrinsic viscosity of less than 0.1 dL/g, degradation of the polymer is too fast, which may make it difficult to sustained-release the GLP-1 RA-based peptides until a desired time, and if it has an intrinsic viscosity exceeding 1.9 dL/g, degradation of the polymer is too slow, and thus, the release amount of the GLP-1 RA-based peptides is small, which may result in a failure to exhibit medicinal effect.

In one embodiment of the present invention, the biocompatible polymer may be included in an amount of 50% to 99.9% by weight based on the total weight of the microspheres. More specifically, the biocompatible polymer may be included in an amount of 50% by weight or more, 55% by weight or more, or 60% by weight or more, or may be included in an amount of 99.9% by weight or less, 99.0% by weight or less, 98.0% by weight or less, 97.0% by weight or less, 96.0% by weight or less, 95.0% by weight or less, 94.0% by weight or less, 93.0% by weight or less, 92.0% by weight or less, 91.0% by weight or less, or 90.0% by weight or less, based on the total weight of the microparticles, but is not limited to these ranges.

If the biocompatible polymer is included in an amount of less than 50% by weight based on the total weight of the microspheres, there may be a problem that the distribution of the GLP-1 RA-based peptides relatively increases, leading to an initial burst release or failure to maintain the medicinal effect for a desired period, and if the biocompatible polymer is included in an amount exceeding 99.9% by weight based on the total weight of the microspheres, the dissolution of the drug may become too slow, making it difficult to administer the drug at a constant blood level.

In one embodiment of the present invention, the microspheres comprising the bioavailability enhancer may sustain the release of the encapsulated GLP-1 RA-based peptides for 20 days or more in an *in vivo* or *in vitro* environment.

In one embodiment of the present invention, a method for preparing microspheres comprising a bioavailability enhancer may comprise the steps of:
(a1) dissolving a biocompatible polymer in one or more solvents to prepare an oil phase (O);
(b1) dissolving or dispersing GLP-1 RA-based peptides and a bioavailability enhancer in a solvent to prepare an inner water phase (W1);
(c1) dispersing the inner water phase (W1) into the oil phase (O) to prepare a water-in-oil (W1/O) type emulsion;
(d1) solidifying microspheres by dispersing the water-in-oil (W1/O) type dispersed phase prepared in the step (c1) into an outer continuous phase (W2) to prepare a water-in-oil-in-water (W1/O/W2) type emulsion solution; and
(e1) removing the solvent.

In one embodiment of the present invention, a method for preparing microspheres comprising a bioavailability enhancer may comprise the steps of:
(a2) dissolving a biocompatible polymer and a bioavailability enhancer in one or more solvents to prepare an oil phase (O);
(b2) dissolving or dispersing GLP-1 RA-based peptides in a solvent to prepare an inner water phase (W1);
(c2) dispersing the inner water phase (W1) into the oil phase (O) to prepare a water-in-oil (W1/O) type emulsion;
(d2) solidifying microspheres by dispersing the water-in-oil (W1/O) type dispersed phase prepared in the step (c2) into an outer continuous phase (W2) to prepare a water-in-oil-in-water (W1/O/W2) type emulsion solution; and
(e2) removing the solvent.

In one embodiment of the present invention, a method for preparing microspheres comprising a bioavailability enhancer may comprise the steps of:
(a3) dissolving a biocompatible polymer and a bioavailability enhancer in one or more solvents to prepare an oil phase (O);
(b3) dissolving or dispersing GLP-1 RA-based peptides and a bioavailability enhancer in a solvent to prepare an inner water phase (W1);
(c3) dispersing the inner water phase (W1) into the oil phase (O) to prepare a water-in-oil (W1/O) type emulsion;
(d3) solidifying microspheres by dispersing the water-in-oil (W1/O) type dispersed phase prepared in the step (c3) into an outer continuous phase (W2) to prepare a water-in-oil-in-water (W1/O/W2) type emulsion solution; and
(e3) removing the solvent.

In one embodiment of the present invention, the bioavailability enhancer may be included in the inner water phase (W1), the oil phase (O), or both the inner water phase (W1) and the oil phase (O) to be prepared into microspheres, and the route and method of inclusion in the microspheres are not particularly limited.

The description of the bioavailability enhancer applies equally as described above.

In one embodiment of the present invention, the solvent used in the steps (a1), (a2) and (a3) is not particularly limited as long as it can dissolve the biocompatible polymer, and preferably, may be a non-aqueous solvent. Non-limiting examples of the non-aqueous solvent may include dichloromethane, chloroform, acetonitrile, dimethyl sulfoxide, dimethylformamide, ethyl acetate, and the like, but are not limited to these examples.

In one embodiment of the present invention, the solvent of the inner water phase (W1) used in the steps (b1), (b2) and (b3) may be any one of distilled water, ethanol, purified water, and combinations thereof.

In one embodiment of the present invention,
in the steps (d1), (d2) and (d3), a hydrophilic polymer may be included as a surfactant, and the type thereof is not particularly limited, and any one may be used as long as it can help the dispersed phase comprising the GLP-1 RA-based peptides and the biocompatible polymer to form a stable droplet dispersed phase within the outer water phase (W2). The hydrophilic polymer may preferably be selected from the group consisting of methylcellulose, polyvinylpyrrolidone, carboxymethylcellulose, lecithin, gelatin, polyvinyl alcohol, polyoxyethylene-polyoxypropylene block copolymer, polyoxyethylene sorbitan fatty acid ester and polyoxyethylene castor oil derivative, and mixtures thereof, and most preferably, may be polyvinyl alcohol.

In the steps (d1), (d2) and (d3), the outer water phase (W2) may be an aqueous hydrophilic polymer solution of 0.1 to 6% (w/w), wherein the molecular weight of the hydrophilic polymer may be 7,000 to 40,000, and the degree of hydrolysis may be 80 to 90%.

In the steps (d1), (d2) and (d3), the dispersed phase containing the GLP-1 RA-based peptides and the hydrophilic polymer prepared in the steps (c1), (c2) and (c3) is added to the outer water phase (W2) containing the hydrophilic polymer in a drop-by-drop manner or in a manner using an in-line mixer, and vigorously stirred to prepare an emulsion solution (W1/O/W2). In these processes, the GLP-1 RA-based peptides are encapsulated into biocompatible polymer microspheres.

Subsequently, in the steps (e1), (e2) and (e3), the solvent is removed, and conventional filtration and washing are performed, and then the desired microspheres may be obtained. That is, a step of washing the obtained microspheres may be included to improve an initial burst suppression effect as needed.

Of the descriptions regarding the microspheres described above, all matters commensurate with the preparation method (the type of biocompatible polymer, intrinsic viscosity thereof, etc.) may be equally applied to the method for preparing the microspheres of the present invention. Therefore, description of redundant contents will be omitted hereinafter.

The present invention may provide a pharmaceutical composition for preventing or treating obesity, diabetes, hypertension, hyperlipidemia, non-alcoholic steatohepatitis, metabolic dysfunction-associated steatohepatitis, cardiovascular disease, or neurodegenerative disease, comprising the microspheres. In this case, the diabetes may be type II diabetes.

The pharmaceutical composition according to the present invention may be formulated into various forms of preparations, and for example, may be formulated into preparations for parenteral administration. The preparations for parenteral administration may be may be formulated in the form of, for example, injections, creams, lotions, ointments for external use, oils, moisturizers, aerosols, patches, and nasal inhalers, by methods known in the art. These formulations are described in the literature (Remington's Pharmaceutical Sciences, 19th ed., Mack Publishing Company, Easton, PA, 1995), which is a prescription book generally known to all pharmaceutical chemistry.

The pharmaceutical composition according to the present invention may be administered to mammals, including humans, by any method. For example, it may be administered parenterally. Parenteral administration methods may include, but are not limited to, intravenous, intramuscular, intra-arterial, intramedullary, intrathecal, intracardiac, transdermal, subcutaneous, intraperitoneal, intranasal, enteral, topical, sublingual, or rectal administration. More specifically, the pharmaceutical composition according to the present invention may be used as a subcutaneous or intramuscular injection.

The formulations for parenteral administration may comprise microspheres alone, or may further comprise excipients, diluents, and/or pharmaceutically acceptable carriers in addition to the microspheres. The carriers include all types of solvents, dispersion media, oil-in-water type or water-in-oil type emulsions, aqueous compositions, liposomes, microbeads, and microsomes.

The pharmaceutically acceptable carriers may further include, for example, carriers for parenteral administration. The carriers for parenteral administration may include water, suitable oils, saline, aqueous glucose, glycol, and the like, and may further include stabilizers and preservatives. Suitable stabilizers include antioxidants such as sodium bisulfite, sodium sulfite, or ascorbic acid. Suitable preservatives include benzalkonium chloride, methyl- or propyl-paraben, and chlorobutanol. The pharmaceutical composition of the present invention may further comprise a lubricant, a wetting agent, a sweetener, a flavoring agent, an emulsifier, a suspending agent, and the like, in addition to the ingredients as described above. Other pharmaceutically acceptable carriers and preparations may be referenced from those described in the literature (Remington's Pharmaceutical Sciences, 19th ed., Mack Publishing Company, Easton, PA, 1995).

The total effective amount of the pharmaceutical composition according to the present invention may be administered to a patient in a single dose, or may administered by a fractionated treatment protocol in which multiple doses are administered over a long period of time. The pharmaceutical composition of the present invention may vary the content of the active ingredient depending on the severity of the disease. The preferred total dose of the pharmaceutical composition of the present invention may be about 0.01 µg to 10,000 mg, most preferably 0.1 µg to 500 mg, per 1 kg of patient body weight per day. However, since the dose of the pharmaceutical composition is determined as an effective dose for a patient considering various factors such as the method of formulation, the route of administration, and the frequency of treatment, as well as the patient's age, body weight, health state, gender, severity of disease, diet, and the rate of excretion, a person having ordinary skill in the art will be able to determine an appropriate effective dose of the composition of the present invention considering these points. The pharmaceutical composition according to the present invention is not particularly limited in its formulation, route of administration, and method of administration, as long as it exhibits the effects of the present invention.

In one embodiment of the present invention, the pharmaceutical composition may be administered to a subject at intervals of one month to one year. The pharmaceutical composition of the present invention may be administered once every two weeks, once every three weeks, once a month, once every three months, once every six months, or once a year, but is not limited thereto.

In one embodiment of the present invention, the pharmaceutical composition may be used for preventing or treating obesity, diabetes, hypertension, hyperlipidemia, non-alcoholic steatohepatitis, metabolic dysfunction-associated steatohepatitis, cardiovascular disease, or neurodegenerative disease.

### [Best Mode]

Hereinafter, the present invention will be described in detail by way of the following examples. However, the following examples are only for illustrating the present invention, and the present invention is not limited by these examples.

### Comparative Examples 1: Preparation of semaglutide microspheres

### (1) Preparation of a continuous phase (900 mL)

900 mL of distilled water (DW) and 4.5 g of polyvinyl alcohol (manufactured by Sigma-Aldrich; the same applies hereinafter) were weighed and placed into a reactor, and 4.5 g of sodium chloride (NaCl) was weighed and placed into the reactor, and then stirred with an overhead stirrer at a speed of 1,000 rpm to prepare a 0.5% polyvinyl alcohol solution.

### (2) Preparation of a discontinuous phase

0.32 g of semaglutide and 2.4 mL of distilled water were weighed, placed into a 50 mL vial, and dissolved by stirring at 150 rpm to prepare Solution A. Subsequently, 2.88 g of PLGA (intrinsic viscosity of 0.1 dL/g to 1.9 dL/g) was weighed and dissolved in 9.6 mL of dichloromethane by stirring at 300 rpm to prepare Solution B.

### (3) Primary homogenization

Solution B obtained in the process of the (2) preparation of a discontinuous phase was taken into a syringe and added to Solution A, and then ground at 20,000 rpm for 1 minute using a homogenizer.

### (4) Preparation of microspheres

After the (3) primary homogenization process was completed, a solution was immediately taken into a syringe, and a homogenizer was connected to the reactor, and then the solution was injected for 60 seconds. Subsequently, after solidifying for 20 hours, microspheres were obtained using a 5 µm filter. The obtained microspheres were freeze-dried in a freeze-dryer for 20 hours or more.

### Comparative Examples 2: Preparation of semaglutide microspheres

### (1) Preparation of a continuous phase (900 mL)

900 ml of distilled water, 4.5 g of polyvinyl alcohol, and 4.5 g of sodium chloride were weighed and placed into a reactor. Subsequently, a 0.5% (w/v) aqueous polyvinyl alcohol solution was prepared by stirring at 1,000 rpm using an overhead stirrer (IKA).

### (2) Preparation of a discontinuous phase

0.2 g of semaglutide and 2.4 ml of distilled water were weighed, placed into a 50 ml vial, and stirred at 150 rpm to prepared Solution A. Subsequently, 1.8 g of PLGA (intrinsic viscosity of 0.1 dL/g to 1.9 dL/g) and 7.2 ml of dichloromethane were placed into another 50 ml vial and stirred at 300 rpm to prepare Solution B.

### (3) Primary homogenization

Solution B obtained in the preparation of primary discontinuous phase was taken into a syringe and added to Solution A, and then stirred at 20,000 rpm for 1 minute using a homogenizer (IKA).

### (4) Preparation of microspheres

After the (3) primary homogenization process was completed, a solution was immediately taken into a syringe, and a homogenizer was connected to the reactor, and then the solution was injected for 60 seconds. Subsequently, after solidifying for 20 hours, microspheres were obtained using a 5 µm filter. The obtained microspheres were freeze-dried in a freeze-dryer for 20 hours or more.

### Comparative Examples 3: Preparation of semaglutide microspheres

### (1) Preparation of a continuous phase (900 mL)

900 ml of distilled water, 4.5 g of polyvinyl alcohol, and 4.5 g of sodium chloride were weighed and placed into a reactor. Subsequently, a 0.5% (w/v) aqueous polyvinyl alcohol solution was prepared by stirring at 1,000 rpm using an overhead stirrer (IKA).

### (2) Preparation of a discontinuous phase

0.08 g of semaglutide and 0.6 ml of distilled water were weighed, placed into a 50 ml vial, and stirred at 150 rpm to prepared Solution A. Subsequently, 0.72 g of PLGA (intrinsic viscosity of 0.1 dL/g to 1.9 dL/g) and 2.4 ml of dichloromethane were placed into another 50 ml vial and stirred at 300 rpm to prepare Solution B.

### (3) Primary homogenization

Solution B obtained in the preparation of primary discontinuous phase was taken into a syringe and added to Solution A, and then stirred at 20,000 rpm for 1 minute using a homogenizer (IKA).

### (4) Preparation of microspheres

After the (3) primary homogenization process was completed, a solution was immediately taken into a syringe, and a homogenizer was connected to the reactor, and then the solution was injected for 60 seconds. Subsequently, after solidifying for 20 hours, microspheres were obtained using a 5 µm filter. The obtained microspheres were freeze-dried in a freeze-dryer for 20 hours or more.

### Example 1: Preparation of microspheres comprising semaglutide and poloxamer

### (1) Preparation of a continuous phase (900 mL)

900 mL of distilled water (DW) and 4.5 g of polyvinyl alcohol were weighed and placed into a reactor, and 4.5 g of sodium chloride (NaCl) was weighed and placed into the reactor, and then stirred with an overhead stirrer at a speed of 1,000 rpm to prepare a 0.5% polyvinyl alcohol solution.

### (2) Preparation of a discontinuous phase

0.32 g of semaglutide was weighed, placed into a 50 mL vial, and suspended in 2.4 mL of distilled water, and then 0.01 g of poloxamer 188 was added thereto and dissolved by stirring at 150 rpm to prepare Solution A. Subsequently, 2.88 g of PLGA (intrinsic viscosity of 0.1 dL/g to 1.9 dL/g) was weighed and dissolved in 9.6 mL of dichloromethane by stirring at 300 rpm to prepare solution B.

### (3) Primary homogenization

Solution B obtained in the process of the (2) preparation of a discontinuous phase was taken into a syringe and added to Solution A, and then ground at 20,000 rpm for 1 minute using a homogenizer.

### (4) Preparation of microspheres

After the (3) primary homogenization process was completed, a solution was immediately taken into a syringe, and a homogenizer was connected to the reactor, and then the solution was injected for 60 seconds. Subsequently, after solidifying for 20 hours, microspheres were obtained using a 5 µm filter. The obtained microspheres were freeze-dried in a freeze-dryer for 20 hours or more.

### Example 2: Preparation of microspheres comprising semaglutide and poloxamer

### (1) Preparation of a continuous phase (900 mL)

900 mL of distilled water (DW) and 4.5 g of polyvinyl alcohol were weighed and placed into a reactor, and 4.5 g of sodium chloride (NaCl) was weighed and placed into the reactor, and then stirred with an overhead stirrer at a speed of 1,000 rpm to prepare a 0.5% polyvinyl alcohol solution.

### (2) Preparation of a discontinuous phase

0.32 g of semaglutide was weighed, placed into a 50 mL vial, and suspended in 2.4 mL of distilled water, and then 0.1 g of poloxamer 188 was added thereto and dissolved by stirring at 150 rpm to prepare Solution A. Subsequently, 2.88 g of PLGA (intrinsic viscosity of 0.1 dL/g to 1.9 dL/g) was weighed and dissolved in 9.6 mL of dichloromethane by stirring at 300 rpm to prepare Solution B.

### (3) Primary homogenization

Solution B obtained in the process of the (2) preparation of a discontinuous phase was taken into a syringe and added to Solution A, and then ground at 20,000 rpm for 1 minute using a homogenizer.

### (4) Preparation of microspheres

After the (3) primary homogenization process was completed, a solution was immediately taken into a syringe, and a homogenizer was connected to the reactor, and then the solution was injected for 60 seconds. Subsequently, after solidifying for 20 hours, microspheres were obtained using a 5 µm filter. The obtained microspheres were freeze-dried in a freeze-dryer for 20 hours or more.

### Example 3: Preparation of microspheres comprising semaglutide and poloxamer

### (1) Preparation of a continuous phase (900 mL)

900 mL of distilled water (DW) and 4.5 g of polyvinyl alcohol were weighed and placed into a reactor, and 4.5 g of sodium chloride (NaCl) was weighed and placed into the reactor, and then stirred with an overhead stirrer at a speed of 1,000 rpm to prepare a 0.5% polyvinyl alcohol solution.

### (2) Preparation of a discontinuous phase

0.32 g of semaglutide was weighed, placed into a 20 mL vial, and suspended in 2.4 mL of distilled water, and then dissolved by stirring at 150 rpm to prepare Solution A. Subsequently, 2.88 g of PLGA (intrinsic viscosity of 0.1 dL/g to 1.9 dL/g) was weighed and suspended in 9.6 mL of dichloromethane, and then 0.1 g of poloxamer 188 was added thereto and dissolved by stirring at 300 rpm to prepare Solution B.

### (3) Primary homogenization

Solution B obtained in the process of the (2) preparation of a discontinuous phase was taken into a syringe and added to Solution A, and then ground at 20,000 rpm for 1 minute using a homogenizer.

### (4) Preparation of microspheres

After the (3) primary homogenization process was completed, a solution was immediately taken into a syringe, and a homogenizer was connected to the reactor, and then the solution was injected for 60 seconds. Subsequently, after solidifying for 20 hours, microspheres were obtained using a 5 µm filter. The obtained microspheres were freeze-dried in a freeze-dryer for 20 hours or more.

### Example 4: Preparation of microspheres comprising semaglutide and propylene glycol

### (1) Preparation of a continuous phase (900 mL)

900 mL of distilled water (DW) and 4.5 g of polyvinyl alcohol were weighed and placed into a reactor, and 4.5 g of sodium chloride (NaCl) was weighed and placed into the reactor, and then stirred with an overhead stirrer at a speed of 1,000 rpm to prepare a 0.5% polyvinyl alcohol solution.

### (2) Preparation of a discontinuous phase

0.32 g of semaglutide was weighed, placed into a 50 mL vial, and suspended in 2.4 mL of distilled water, and then 0.03 mL of propylene glycol was added thereto and dissolved by stirring at 150 rpm to prepare Solution A. Subsequently, 2.88 g of PLGA (intrinsic viscosity of 0.1 dL/g to 1.9 dL/g) was weighed and dissolved in 9.6 mL of dichloromethane by stirring at 300 rpm to prepare solution B.

### (3) Primary homogenization

Solution B obtained in the process of the (2) preparation of a discontinuous phase was taken into a syringe and added to Solution A, and then ground at 20,000 rpm for 1 minute using a homogenizer.

### (4) Preparation of microspheres

After the (3) primary homogenization process was completed, a solution was immediately taken into a syringe, and a homogenizer was connected to a reactor, and then the solution was injected for 60 seconds. Subsequently, after solidifying for 20 hours, microspheres were obtained using a 5 µm filter. The obtained microspheres were freeze-dried in a freeze-dryer for 20 hours or more.

### Example 5: Preparation of microspheres comprising semaglutide and propylene glycol

### (1) Preparation of a continuous phase (900 mL)

900 mL of distilled water (DW) and 4.5 g of polyvinyl alcohol were weighed and placed into a reactor, and 4.5 g of sodium chloride (NaCl) was weighed and placed into the reactor, and then stirred with an overhead stirrer at a speed of 1,000 rpm to prepare a 0.5% polyvinyl alcohol solution.

### (2) Preparation of a discontinuous phase

0.32 g of semaglutide was weighed, placed into a 50 mL vial, and suspended in 2.4 mL of distilled water, and then 0.03 mL of propylene glycol was added thereto and dissolved by stirring at 150 rpm to prepare Solution A. Subsequently, 2.88 g of PLGA (intrinsic viscosity of 0.1 dL/g to 1.9 dL/g) was weighed and suspended in 9.6 mL of dichloromethane, and then 0.03 mL of propylene glycol was added thereto and dissolved by stirring at 300 rpm to prepare Solution B.

### (3) Primary homogenization

Solution B obtained in the process of the (2) preparation of a discontinuous phase was taken into a syringe and added to Solution A, and then ground at 20,000 rpm for 1 minute using a homogenizer.

### (4) Preparation of microspheres

After the (3) primary homogenization process was completed, a solution was immediately taken into a syringe, and a homogenizer was connected to a reactor, and then the solution was injected for 60 seconds. Subsequently, after solidifying for 20 hours, microspheres were obtained using a 5 µm filter. The obtained microspheres were freeze-dried in a freeze-dryer for 20 hours or more.

### Example 6: Preparation of microspheres comprising semaglutide and propylene glycol

### (1) Preparation of a continuous phase (900 mL)

900 mL of distilled water (DW) and 4.5 g of polyvinyl alcohol were weighed and placed into a reactor, and 4.5 g of sodium chloride (NaCl) was weighed and placed into the reactor, and then stirred with an overhead stirrer at a speed of 1,000 rpm to prepare a 0.5% polyvinyl alcohol solution.

### (2) Preparation of a discontinuous phase

0.32 g of semaglutide was weighed, placed into a 20 mL vial, and suspended in 2.4 mL of distilled water, and then 0.09 mL of propylene glycol was added thereto and dissolved by stirring at 150 rpm to prepare Solution A. Subsequently, 2.88 g of PLGA (intrinsic viscosity of 0.1 dL/g to 1.9 dL/g) was weighed and dissolved in 9.6 mL of dichloromethane by stirring at 300 rpm to prepare Solution B.

### (3) Primary homogenization

Solution B obtained in the process of the (2) preparation of a discontinuous phase was taken into a syringe and added to Solution A, and then ground at 20,000 rpm for 1 minute using a homogenizer.

### (4) Preparation of microspheres

After the (3) primary homogenization process was completed, a solution was immediately taken into a syringe, and a homogenizer was connected to the reactor, and then the solution was injected for 30 seconds. Subsequently, after solidifying for 20 hours, microspheres were obtained using a 5 µm filter. The obtained microspheres were freeze-dried in a freeze-dryer for 20 hours or more.

### Example 7: Preparation of microspheres comprising semaglutide and polyoxyethylene oleyl ether

### (1) Preparation of a continuous phase (900 mL)

900 mL of distilled water (DW) and 4.5 g of polyvinyl alcohol were weighed and placed into a reactor, and 4.5 g of sodium chloride (NaCl) was weighed and placed into the reactor, and then stirred with an overhead stirrer at a speed of 1,000 rpm to prepare a 0.5% polyvinyl alcohol solution.

### (2) Preparation of a discontinuous phase

0.32 g of semaglutide was weighed, placed into a 20 mL vial, and suspended in 2.4 mL of distilled water, and then dissolved by stirring at 150 rpm to prepare Solution A. Subsequently, 2.88 g of PLGA (intrinsic viscosity of 0.1 dL/g to 1.9 dL/g) and 9.6 mL of dichloromethane were weighed and added thereto, and then 0.11 mL of polyoxyethylene oleyl ether (Brij^{®} 98) was added thereto and dissolved by stirring at 300 rpm to prepare Solution B.

### (3) Primary homogenization

Solution B obtained in the process of the (2) preparation of a discontinuous phase was taken into a syringe and added to Solution A, and then ground at 20,000 rpm for 1 minute using a homogenizer.

### (4) Preparation of microspheres

After the (3) primary homogenization process was completed, a solution was immediately taken into a syringe, and a homogenizer was connected to the reactor, and then the solution was injected for 60 seconds. Subsequently, after solidifying for 20 hours, microspheres were obtained using a 5 µm filter. The obtained microspheres were freeze-dried in a freeze-dryer for 20 hours or more.

### Example 8: Preparation of microspheres comprising semaglutide and lysine

### (1) Preparation of a continuous phase (900 mL)

900 mL of distilled water (DW) and 4.5 g of polyvinyl alcohol were weighed and placed into a reactor, and 4.5 g of sodium chloride (NaCl) was weighed and placed into the reactor, and then stirred with an overhead stirrer at a speed of 1,000 rpm to prepare a 0.5% polyvinyl alcohol solution.

### (2) Preparation of a discontinuous phase

0.2 g of semaglutide was weighed, placed into a 50 mL vial, and suspended in 2.4 mL of distilled water, and then 12 mg of lysine was added thereto and dissolved by stirring at 150 rpm to prepare Solution A. Subsequently, 1.8 g of PLGA (intrinsic viscosity of 0.1 dL/g to 1.9 dL/g) was weighed and dissolved in 7.2 mL of dichloromethane by stirring at 300 rpm to prepare Solution B.

### (3) Primary homogenization

Solution B obtained in the process of the (2) preparation of a discontinuous phase was taken into a syringe and added to Solution A, and then ground at 20,000 rpm for 1 minute using a homogenizer.

### (4) Preparation of microspheres

After the (3) primary homogenization process was completed, a solution was immediately taken into a syringe, and a homogenizer was connected to the reactor, and then the solution was injected for 60 seconds. Subsequently, after solidifying for 20 hours, microspheres were obtained using a 5 µm filter. The obtained microspheres were freeze-dried in a freeze-dryer for 20 hours or more.

### Example 9: Preparation of microspheres comprising semaglutide and lysine

### (1) Preparation of a continuous phase (900 mL)

900 mL of distilled water (DW) and 4.5 g of polyvinyl alcohol were weighed and placed into a reactor, and 4.5 g of sodium chloride (NaCl) was weighed and placed into the reactor, and then stirred with an overhead stirrer at a speed of 1,000 rpm to prepare a 0.5% polyvinyl alcohol solution.

### (2) Preparation of a discontinuous phase

0.2 g of semaglutide was weighed, placed into a 50 mL vial, and suspended in 2.4 mL of distilled water, and then 24 mg of lysine was added thereto and dissolved by stirring at 150 rpm to prepare Solution A. Subsequently, 1.8 g of PLGA (intrinsic viscosity of 0.1 dL/g to 1.9 dL/g) was weighed and dissolved in 7.2 mL of dichloromethane by stirring at 300 rpm to prepare Solution B.

### (3) Primary homogenization

Solution B obtained in the process of the (2) preparation of a discontinuous phase was taken into a syringe and added to Solution A, and then ground at 20,000 rpm for 1 minute using a homogenizer.

### (4) Preparation of microspheres

After the (3) primary homogenization process was completed, a solution was immediately taken into a syringe, and a homogenizer was connected to the reactor, and then the solution was injected for 60 seconds. Subsequently, after solidifying for 20 hours, microspheres were obtained using a 5 µm filter. The obtained microspheres were freeze-dried in a freeze-dryer for 20 hours or more.

### Example 10: Preparation of microspheres comprising semaglutide and lysine

### (1) Preparation of a continuous phase (900 mL)

900 mL of distilled water (DW) and 4.5 g of polyvinyl alcohol were weighed and placed into a reactor, and 4.5 g of sodium chloride (NaCl) was weighed and placed into the reactor, and then stirred with an overhead stirrer at a speed of 1,000 rpm to prepare a 0.5% polyvinyl alcohol solution.

### (2) Preparation of a discontinuous phase

0.2 g of semaglutide was weighed, placed into a 50 mL vial, and suspended in 2.4 mL of distilled water, and then 36 mg of lysine was added thereto and dissolved by stirring at 150 rpm to prepare Solution A. Subsequently, 1.8 g of PLGA (intrinsic viscosity of 0.1 dL/g to 1.9 dL/g) was weighed and dissolved in 7.2 mL of dichloromethane by stirring at 300 rpm to prepare Solution B.

### (3) Primary homogenization

Solution B obtained in the process of the (2) preparation of a discontinuous phase was taken into a syringe and added to Solution A, and then ground at 20,000 rpm for 1 minute using a homogenizer.

### (4) Preparation of microspheres

After the (3) primary homogenization process was completed, a solution was immediately taken into a syringe, and a homogenizer was connected to the reactor, and then the solution was injected for 60 seconds. Subsequently, after solidifying for 20 hours, microspheres were obtained using a 5 µm filter. The obtained microspheres were freeze-dried in a freeze-dryer for 20 hours or more.

### Example 11: Preparation of microspheres comprising semaglutide and arginine

### (1) Preparation of a continuous phase (900 mL)

900 mL of distilled water (DW) and 4.5 g of polyvinyl alcohol were weighed and placed into a reactor, and 4.5 g of sodium chloride (NaCl) was weighed and placed into the reactor, and then stirred with an overhead stirrer at a speed of 1,000 rpm to prepare a 0.5% polyvinyl alcohol solution.

### (2) Preparation of a discontinuous phase

0.16 g of semaglutide was weighed, placed into a 50 mL vial, and suspended in 1.2 mL of distilled water, and then 80 mg of arginine was added thereto and dissolved by stirring at 150 rpm to prepare Solution A. Subsequently, 1.44 g of PLGA (intrinsic viscosity of 0.1 dL/g to 1.9 dL/g) was weighed and dissolved in 4.8 mL of dichloromethane by stirring at 300 rpm to prepare Solution B.

### (3) Primary homogenization

Solution B obtained in the process of the (2) preparation of a discontinuous phase was taken into a syringe and added to Solution A, and then ground at 7,600 rpm for 1 minute using a homogenizer.

### (4) Preparation of microspheres

After the (3) primary homogenization process was completed, a solution was immediately taken into a syringe, and a homogenizer was connected to the reactor, and then the solution was injected for 60 seconds. Subsequently, after solidifying for 20 hours, microspheres were obtained using a 5 µm filter. The obtained microspheres were freeze-dried in a freeze-dryer for 20 hours or more.

### Example 12: Preparation of microspheres comprising semaglutide and stearic acid

### (1) Preparation of a continuous phase (900 mL)

900 mL of distilled water (DW) and 4.5 g of polyvinyl alcohol were weighed and placed into a reactor, and 4.5 g of sodium chloride (NaCl) was weighed and placed into the reactor, and then stirred with an overhead stirrer at a speed of 1,000 rpm to prepare a 0.5% polyvinyl alcohol solution.

### (2) Preparation of a discontinuous phase

0.1 g of semaglutide was weighed, placed into a 50 mL vial, and suspended in 1.2 mL of distilled water, and then dissolved by stirring at 150 rpm to prepare Solution A. Subsequently, 0.9 g of PLGA (intrinsic viscosity of 0.1 dL/g to 1.9 dL/g) and 6 mg of stearic acid were weighed and dissolved in 3.6 mL of dichloromethane by stirring at 300 rpm to prepare Solution B.

### (3) Primary homogenization

Solution B obtained in the process of the (2) preparation of a discontinuous phase was taken into a syringe and added to Solution A, and then ground at 20,000 rpm for 1 minute using a homogenizer.

### (4) Preparation of microspheres

After the (3) primary homogenization process was completed, a solution was immediately taken into a syringe, and a homogenizer was connected to the reactor, and then the solution was injected for 60 seconds. Subsequently, after solidifying for 20 hours, microspheres were obtained using a 5 µm filter. The obtained microspheres were freeze-dried in a freeze-dryer for 20 hours or more.

### Experimental Example 1: Evaluation of loading rate and initial release rate of microspheres

To evaluate the loading rate of the microspheres, the microspheres prepared in the Comparative Examples 1 to 3 and Examples 1 to 12 were taken and placed into a 20 mL volumetric flask, and completely dissolved in acetonitrile containing 0.1% trifluoroacetic acid (TFA), and then adjusted to the graduation mark with distilled water containing 0.1% trifluoroacetic acid (TFA) and filtered through a 0.45 µm RC filter. This solution was analyzed using HPLC with an ultraviolet-visible spectrophotometric detector.

To evaluate the initial release rate of the microspheres, the microspheres prepared in Comparative Examples 1 to 3 and Examples 1 to 12 above were taken and placed into a vial, a pH 7.4 PBS solution was added thereto, and the mixture was maintained at 37°C by stirring at 100 rpm. To measure the release amount over a specific period of time, the supernatant was taken after centrifugation and filtered through a 0.45 µm RC filter. This solution was placed into a vial and analyzed using HPLC with an ultraviolet-visible spectrophotometer. The experimental results are shown in Table 1 below.

**[Table 1]**

| Classification | Loading rate (%) | Initial release rate (%) |
|---|---|---|
| Comparative Example 1 | 10.5 | 0.6 |
| Comparative Example 2 | 10.2 | 0.8 |
| Comparative Example 3 | 10.4 | 2.1 |
| Example 1 | 10.2 | 2.7 |
| Example 2 | 9.64 | 1.7 |
| Example 3 | 9.90 | 2.0 |
| Example 4 | 10.6 | 2.8 |
| Example 5 | 10.6 | 0.9 |
| Example 6 | 10.3 | 0.7 |
| Example 7 | 10.3 | 3.3 |
| Example 8 | 9.8 | 1.0 |
| Example 9 | 9.5 | 3.4 |
| Example 10 | 9.3 | 7.2 |
| Example 12 | 10.0 | 0.9 |

### Experimental Example 2: In vivo pharmacokinetic test using beagles

After the injectable semaglutide microsphere formulations of the Comparative Example 3 and Examples 6 to 10 were subcutaneously injected into beagles, blood was collected at designated time points, and the blood concentration of semaglutide was measured using LC-MS/MS. The area under the curve (AUC) was calculated from a blood concentration-time graph of semaglutide. The experimental results are shown in Table 2 below.

**[Table 2]**

| Classification | AUCₗₐₛₜ (ng*hr/mL) | Rate of increase (%) in area under the curve compared to Comparative Example 3 |
|---|---|---|
| Comparative Example 3 | 31,759.52 | - |
| Example 6 | 115,862.80 | 264.74 |
| Example 7 | 71,801.00 | 126.08 |
| Example 8 | 135,892.80 | 327.74 |
| Example 9 | 192,499.80 | 506.15 |
| Example 10 | 251,885.40 | 692.99 |

As confirmed in Table 2, the microspheres comprising the bioavailability enhancer (Examples 6 to 10) were found to exhibit an increase in the area under the concentration-time curve by at least approximately 126% or more, compared to the microspheres without the addition thereof (Comparative Example 3).

Since the bioavailability is proportional to the area under the curve (AUCₗₐₛₜ) of a blood concentration-time graph, these results indicate that the microspheres of the present invention comprising a bioavailability enhancer provide significantly improved bioavailability.

### Comparative Examples 4: Preparation of tirzepatide microspheres

### (1) Preparation of a continuous phase (900 mL)

900 mL of distilled water (DW) and 4.5 g of polyvinyl alcohol were weighed and placed into a reactor, and 4.5 g of sodium chloride (NaCl) was weighed and placed into the reactor, and then stirred with an overhead stirrer at a speed of 1,000 rpm to prepare a 0.5% polyvinyl alcohol solution.

### (2) Preparation of a discontinuous phase

0.386 g of tirzepatide and 1.5 mL of distilled water were weighed, placed into a 20 mL vial, and dissolved by stirring at 150 rpm to prepare Solution C. Subsequently, 0.9 g of PLGA (intrinsic viscosity of 0.1 dL/g to 1.9 dL/g) was weighed and dissolved in 3.0 mL of dichloromethane by stirring at 300 rpm to prepare Solution D.

### (3) Primary homogenization

Solution D obtained in the process of the (2) preparation of a discontinuous phase was taken into a syringe and added to Solution C, and then ground at 20,000 rpm for 1 minute using a homogenizer.

### (4) Preparation of microspheres

After the (3) primary homogenization process was completed, a solution was immediately taken into a syringe, and a homogenizer was connected to the reactor, and then the solution was injected for 60 seconds. Subsequently, after solidifying for 20 hours, microspheres were obtained using a 5 µm filter. The obtained microspheres were freeze-dried in a freeze-dryer for 20 hours or more.

### Example 13: Preparation of microspheres comprising tirzepatide and poloxamer

### (1) Preparation of a continuous phase (900 mL)

900 mL of distilled water (DW) and 4.5 g of polyvinyl alcohol were weighed and placed into a reactor, and 4.5 g of sodium chloride (NaCl) was weighed and placed into the reactor, and then stirred with an overhead stirrer at a speed of 1,000 rpm to prepare a 0.5% polyvinyl alcohol solution.

### (2) Preparation of a discontinuous phase

0.386 g of tirzepatide and 1.5 mL of distilled water were weighed, placed into a 20 mL vial, and dissolved by stirring at 150 rpm to prepare Solution C. Subsequently, 0.9 g of PLGA (intrinsic viscosity of 0.1 dL/g to 1.9 dL/g) was weighed and dissolved in 3.0 mL of dichloromethane by stirring at 300 rpm, and then 0.1 g of poloxamer 188 was added thereto to prepare Solution D.

### (3) Primary homogenization

Solution D obtained in the process of the (2) preparation of a discontinuous phase was taken into a syringe and added to Solution C, and then ground at 20,000 rpm for 1 minute using a homogenizer.

### (4) Preparation of microspheres

After the (3) primary homogenization process was completed, a solution was immediately taken into a syringe, and a homogenizer was connected to the reactor, and then the solution was injected for 60 seconds. Subsequently, after solidifying for 20 hours, microspheres were obtained using a 5 µm filter. The obtained microspheres were freeze-dried in a freeze-dryer for 20 hours or more.

### Example 14: Preparation of microspheres comprising tirzepatide and propylene glycol

### (1) Preparation of a continuous phase (900 mL)

900 mL of distilled water (DW) and 4.5 g of polyvinyl alcohol were weighed and placed into a reactor, and 4.5 g of sodium chloride (NaCl) was weighed and placed into the reactor, and then stirred with an overhead stirrer at a speed of 1,000 rpm to prepare a 0.5% polyvinyl alcohol solution.

### (2) Preparation of a discontinuous phase

0.386 g of tirzepatide and 1.5 mL of distilled water were weighed and placed into a 20 mL vial, and then stirred at 150 rpm to prepare Solution E. Subsequently, 0.9 g of PLGA (intrinsic viscosity of 0.1 dL/g to 1.9 dL/g) was weighed and dissolved in 3.0 mL of dichloromethane by stirring at 300 rpm, and then 0.11 mL of propylene glycol was added thereto and stirred to prepare Solution F.

### (3) Primary homogenization

Solution F obtained in the process of the (2) preparation of a discontinuous phase was taken into a syringe and added to Solution E, and then ground at 20,000 rpm for 1 minute using a homogenizer.

### (4) Preparation of microspheres

After the (3) primary homogenization process was completed, a solution was immediately taken into a syringe, and a homogenizer was connected to the reactor, and then the solution was injected for 60 seconds. Subsequently, after solidifying for 20 hours, microspheres were obtained using a 5 µm filter. The obtained microspheres were freeze-dried in a freeze-dryer for 20 hours or more.

### Example 15: Preparation of microspheres comprising tirzepatide and propylene glycol

### (1) Preparation of a continuous phase (900 mL)

900 mL of distilled water (DW) and 4.5 g of polyvinyl alcohol were weighed and placed into a reactor, and 4.5 g of sodium chloride (NaCl) was weighed and placed into the reactor, and then stirred with an overhead stirrer at a speed of 1,000 rpm to prepare a 0.5% polyvinyl alcohol solution.

### (2) Preparation of a discontinuous phase

0.386 g of tirzepatide and 1.5 mL of distilled water were weighed, placed into a 20 mL vial, and dissolved by stirring at 150 rpm, and then 0.11 mL of propylene glycol was added thereto and stirred at 150 rpm to prepare Solution E. Subsequently, 0.9 g of PLGA (intrinsic viscosity of 0.1 dL/g to 1.9 dL/g) was weighed and dissolved in 3.0 mL of dichloromethane by stirring at 300 rpm to prepare Solution F.

### (3) Primary homogenization

Solution F obtained in the process of the (2) preparation of a discontinuous phase was taken into a syringe and added to Solution E, and then ground at 20,000 rpm for 1 minute using a homogenizer.

### (4) Preparation of microspheres

After the (3) primary homogenization process was completed, a solution was immediately taken into a syringe, and a homogenizer was connected to the reactor, and then the solution was injected for 60 seconds. Subsequently, after solidifying for 20 hours, microspheres were obtained using a 5 µm filter. The obtained microspheres were freeze-dried in a freeze-dryer for 20 hours or more.

### Example 16: Preparation of microspheres comprising tirzepatide, lysine, and propylene glycol

### (1) Preparation of a continuous phase (900 mL)

900 mL of distilled water (DW) and 4.5 g of polyvinyl alcohol were weighed and placed into a reactor, and 4.5 g of sodium chloride (NaCl) was weighed and placed into the reactor, and then stirred with an overhead stirrer at a speed of 1,000 rpm to prepare a 0.5% polyvinyl alcohol solution.

### (2) Preparation of a discontinuous phase

0.321 g of tirzepatide, 1.2 mL of distilled water, 2.4 mg of lysine, and 0.031 mL of propylene glycol were weighed, placed into a 20 mL vial, and dissolved by stirring at 150 rpm to prepare Solution C. Subsequently, 0.75 g of PLGA (intrinsic viscosity of 0.1 dL/g to 1.9 dL/g) was weighed and dissolved in 3.0 mL of dichloromethane by stirring at 300 rpm to prepare Solution D.

### (3) Primary homogenization

Solution D obtained in the process of the (2) preparation of a discontinuous phase was taken into a syringe and added to Solution C, and then ground at 20,000 rpm for 1 minute using a homogenizer.

### (4) Preparation of microspheres

After the (3) primary homogenization process was completed, a solution was immediately taken into a syringe, and a homogenizer was connected to the reactor, and then the solution was injected for 60 seconds. Subsequently, after solidifying for 20 hours, microspheres were obtained using a 5 µm filter. The obtained microspheres were freeze-dried in a freeze-dryer for 20 hours or more.

### Example 17: Preparation of microspheres comprising tirzepatide, lysine, and propylene glycol

### (1) Preparation of a continuous phase (900 mL)

900 mL of distilled water (DW) and 4.5 g of polyvinyl alcohol were weighed and placed into a reactor, and 4.5 g of sodium chloride (NaCl) was weighed and placed into the reactor, and then stirred with an overhead stirrer at a speed of 1,000 rpm to prepare a 0.5% polyvinyl alcohol solution.

### (2) Preparation of a discontinuous phase

0.321 g of tirzepatide, 1 mL of distilled water, 7 mg of lysine, and 0.031 mL of propylene glycol were weighed, placed into a 20 mL vial, and dissolved by stirring at 150 rpm to prepare Solution C. Subsequently, 0.375 g of PLGA (intrinsic viscosity of 0.1 dL/g to 1.9 dL/g) was weighed and dissolved in 3.0 mL of dichloromethane by stirring at 300 rpm to prepare Solution D.

### (3) Primary homogenization

Solution D obtained in the process of the (2) preparation of a discontinuous phase was taken into a syringe and added to Solution C, and then ground at 20,000 rpm for 1 minute using a homogenizer.

### (4) Preparation of microspheres

After the (3) primary homogenization process was completed, a solution was immediately taken into a syringe, and a homogenizer was connected to the reactor, and then the solution was injected for 60 seconds. Subsequently, after solidifying for 20 hours, microspheres were obtained using a 5 µm filter. The obtained microspheres were freeze-dried in a freeze-dryer for 20 hours or more.

### Experimental Example 3: Evaluation of initial release rate of microspheres

To evaluate the loading rate of the microspheres, the microspheres prepared in Comparative Example 4 and Examples 13 to 17 above were taken and placed into a 20 mL volumetric flask, and completely dissolved in acetonitrile containing 0.1% trifluoroacetic acid (TFA), and then adjusted to the graduation mark with distilled water containing 0.1% trifluoroacetic acid (TFA) and filtered through a 0.45 µm RC filter. This solution was analyzed using HPLC with an ultraviolet-visible spectrophotometric detector.

To evaluate the initial release rate of the microspheres, the microspheres prepared in the Comparative Example 4 and Examples 13 to 17 were placed into a vial, a pH 7.4 PBS solution was added thereto, and the mixture was maintained at 37°C by stirring at 100 rpm. To measure the release amount over a specific period of time, the supernatant was taken after centrifugation and filtered through a 0.45 µm RC filter. This solution was placed into a vial and analyzed using HPLC with an ultraviolet-visible spectrophotometer. The experimental results are shown in Table 3 below.

**[Table 3]**

| Classification | Loading rate (%) | Initial release rate (%) |
|---|---|---|
| Comparative Example 4 | 30.7 | 1.3 |
| Example 13 | 29.7 | 0.6 |
| Example 14 | 26.8 | 2.8 |
| Example 15 | 30.1 | 1.4 |
| Example 16 | 31.0 | 4.1 |
| Example 17 | 28.3 | 1.5 |

### Experimental Example 4: In vivo pharmacokinetic test using beagles

After the injectable tirzepatide microsphere formulations of the Comparative Example 4 and Examples 15 to 17 were subcutaneously injected into beagles, blood was collected at designated time points, and the blood concentration of tirzepatide was measured using LC-MS/MS. The area under the curve (AUC) was calculated from a blood concentration-time graph of tirzepatide.

**[Table 4]**

| | AUCₗₐₛₜ (ng*hr/mL) | Rate of increase (%) in area under the curve compared to Comparative Example 4 |
|---|---|---|
| Comparative Example 4 | 468,991.60 | - |
| Example 15 | 679,774.60 | 44.94 |
| Example 16 | 782,749.20 | 66.90 |
| Example 17 | 1,003,198.50 | 113.91 |

As confirmed in Table 4, the microspheres comprising the bioavailability enhancer (Examples 15 to 17) were found to exhibit an increase in the area under the concentration-time curve by at least approximately 45% or more, compared to the microspheres without the addition thereof (Comparative Example 4).

Since the bioavailability is proportional to the area under the curve (AUCₗₐₛₜ) of a blood concentration-time graph, these results indicate that the microspheres of the present invention comprising a bioavailability enhancer provide significantly improved bioavailability.

## Claims

1. Microspheres comprising:
GLP-1 RA-based peptides;
a bioavailability enhancer; and
a biocompatible polymer.

2. The microspheres according to claim 1, wherein the GLP-1 RA-based peptides are one or more selected from the group consisting of a GLP-1 receptor agonist, a GIP/GLP-1 receptor dual agonist, and a GLP-1/GIP/GCG receptor triple agonist.

3. The microspheres according to claim 1, wherein the bioavailability enhancer is one or more selected from the group consisting of a surfactant, a polyhydric alcohol, an amino acid, and a fatty acid.

4. The microspheres according to claim 1, wherein the bioavailability enhancer is included in an amount of 0.05% to 50% by weight based on the total weight of the microspheres.

5. The microspheres according to claim 3, wherein the surfactant is one or more selected from poloxamer 188, poloxamer 407, polysorbate 20, polysorbate 60, polysorbate 80, polyoxyethylene oleyl ether, polyoxyethylene cetyl ether, polyoxyethylene stearyl ether, and polyoxyethylene lauryl ether.

6. The microspheres according to claim 3, wherein the polyhydric alcohol is one or more selected from ethylene glycol, propylene glycol, polyethylene glycol, glycerol, erythritol, threitol, xylitol, ribitol, mannitol, sorbitol, and maltitol.

7. The microspheres according to claim 3, wherein the amino acid is one or more selected from lysine, arginine, aspartic acid, histidine, alanine, phenylalanine, serine, glutamine, glycine, tryptophan, valine, leucine, isoleucine, cysteine, methionine, and proline.

8. The microspheres according to claim 3, wherein the fatty acid is one or more selected from stearic acid, palmitic acid, lauric acid, myristic acid, oleic acid, and arachidonic acid.

9. The microspheres according to claim 1, wherein the GLP-1 RA-based peptides are selected from semaglutide, tirzepatide, retatrutide, exenatide, liraglutide, lixisenatide, albiglutide, dulaglutide, cotadutide, taspoglutide, pemvidutide, survodutide, orforglipron, mazdutide, MariTide, ecnoglutide, cagrilintide/semaglutide, efpeglenatide, dapiglutide, pramlintide, and combinations thereof.

10. The microspheres according to claim 1, wherein the GLP-1 RA-based peptides are included in an amount of 0.1% to 50% by weight based on the total weight of the microspheres.

11. The microspheres according to claim 1, wherein the biocompatible polymer is any one or more selected from polylactic acid, polylactide, polylactic-co-glycolic acid, polylactide-co-glycolide (PLGA), polyphosphazine, polyiminocarbonate, polyphosphoester, polyanhydride, polyorthoester, copolymer of lactic acid and caprolactone, polycaprolactone, polyhydroxyvalerate, polyhydroxybutyrate, polyamino acid, and copolymer of lactic acid and amino acid.

12. The microspheres according to claim 1, wherein the biocompatible polymer is included in an amount of 50% to 99.9% by weight based on the total weight of the microspheres.

13. A method for preparing microspheres, comprising the steps of:
(a1) dissolving a biocompatible polymer in one or more solvents to prepare an oil phase (O);
(b1) dissolving or dispersing GLP-1 RA-based peptides and a bioavailability enhancer in a solvent to prepare an inner water phase (W1);
(c1) dispersing the inner water phase (W1) into the oil phase (O) to prepare a water-in-oil (W1/O) type emulsion;
(d1) solidifying microspheres by dispersing the water-in-oil (W1/O) type dispersed phase prepared in step (c1) into an outer continuous phase (W2) to prepare a water-in-oil-in-water (W1/O/W2) type emulsion solution; and
(e1) removing the solvent.

14. A method for preparing microspheres, comprising the steps of:
(a2) dissolving a biocompatible polymer and a bioavailability enhancer in one or more solvents to prepare an oil phase (O);
(b2) dissolving or dispersing GLP-1 RA-based peptides in a solvent to prepare an inner water phase (W1);
(c2) dispersing the inner water phase (W1) into the oil phase (O) to prepare a water-in-oil (W1/O) type emulsion;
(d2) solidifying microspheres by dispersing the water-in-oil (W1/O) type dispersed phase prepared in the step (c2) into an outer continuous phase (W2) to prepare a water-in-oil-in-water (W1/O/W2) type emulsion solution; and
(e2) removing the solvent.

15. A method for preparing microspheres, comprising the steps of:
(a3) dissolving a biocompatible polymer and a bioavailability enhancer in one or more solvents to prepare an oil phase (O);
(b3) dissolving or dispersing GLP-1 RA-based peptides and a bioavailability enhancer in a solvent to prepare an inner water phase (W1);
(c3) dispersing the inner water phase (W1) into the oil phase (O) to prepare a water-in-oil (W1/O) type emulsion;
(d3) solidifying microspheres by dispersing the water-in-oil (W1/O) type dispersed phase prepared in the step (c3) into an outer continuous phase (W2) to prepare a water-in-oil-in-water (W1/O/W2) type emulsion solution; and
(e3) removing the solvent.

16. A pharmaceutical composition for preventing or treating obesity, diabetes, hypertension, hyperlipidemia, non-alcoholic steatohepatitis, metabolic dysfunction-associated steatohepatitis, cardiovascular disease, or neurodegenerative disease, comprising the microspheres according to any one of claims 1 to 12.

17. The pharmaceutical composition according to claim 16, wherein the pharmaceutical composition is for subcutaneous or intramuscular injection.
